# EUROPÄISCHE PATENTSCHRIFT

(11) **EP 0 415 113 B1**
(45) Veröffentlichungstag und Bekanntmachung des Hinweises auf die Patenterteilung: **11.01.1995**
(21) Anmeldenummer: 90114818.9
(22) Anmeldetag: 02.08.1990
(51) Int. Cl.: A61B 5/20

(54) **Messeinrichtung zur Durchflussmessung von Körperflüssigkeit**
Flow measurement means of body liquid
Dispositif de mesure de débit pour liquide du corps

(30) Priorität: 11.08.1989 DE 3926630
(43) Veröffentlichungstag der Anmeldung: 06.03.1991
(73) Patentinhaber: Richard Wolf GmbH, 75438 Knittlingen (DE)
(72) Erfinder: Ams, Felix, D-7539 Kämpfelbach (DE); Baier, Manfred, D-7134 Knittlingen (DE); Schäfer, Roland, D-7518 Bretten-Dürrenbüchig (DE)
(74) Vertreter: Vollmann, Heiko, Dipl.-Ing.

(56) Entgegenhaltungen:
- EP-A- 0 077 413
- DE-U- 7 815 730
- FR-A- 2 154 024
- US-A- 4 891 993

## Beschreibung

Die Erfindung betrifft eine Meßeinrichtung zur Durchflußmessung für die urologische Funktionsdiagnostik durch Messung des Harnflusses, mit einem Gefäß zum Auffangen der Urin-Flüssigkeit und mit Mitteln zur Messung der elektrischen Leitfähigkeit derselben.

In der urologischen Funktionsdiagnostik wird häufig eine Messung des Harnflusses (Uroflowmetrie) durchgeführt. Aus der Analyse der Uroflowmetriekurve kann das Maß einer Blasenentleerungsstörung objektiv festgestellt werden. Voraussetzung dafür sind Meßgeräte, die eine hinreichend genaue, fortlaufende und reproduzierbare Aufzeichnung der Meßwerte liefern.

Bekannte Uroflowmeter arbeiten nach verschiedenen Meßprinzipien.Die Messung des Harnflusses und des Miktionsvolumens kann z.B. gravimetrisch, rotationsdynamisch, kapazitiv oder induktiv erfolgen. Bei den gravimetrischen Verfahren wird das Gewicht des Miktionsvolumens gemessen. Hierzu wird der während der Miktion ausgeschiedene Urin in einem Meßzylinder aufgefangen und Gewichtszunahmen laufend gemessen und aufgezeichnet.

Bei der Anwendung des rotationsdynamischen Prinzips trifft der Harnstrahl auf eine um eine senkrechte Achse rotierende Scheibe, wodurch die Scheibe abgebremst wird. Eine elektronische Steuerung sorgt für eine konstante Drehzahl und die hierbei vom Antriebsmotor aufgenommene elektrische Energie ist somit ein Maß für die auftreffende Harnmenge und folglich für den Harnfluß. Der Urin wird auf der Scheibe beschleunigt und schließlich gegen die Wand des Gehäuses geschleudert, das als Auffanggefäß dient.

Die induktive Durchflußmessung macht von der Wirkung eines Magnetfeldes auf bewegte Materie Gebrauch. Wird ein elektrischer Leiter (Urin) im Magnetfeld bewegt, so wird in ihm eine elektrische Spannung induziert. Diese induzierte Spannung ist bei Flüssigkeiten der mittleren Strömungsgeschwindigkeit proportional. Zur Durchflußmessung werden senkrecht zum Magnetfeld in einem nichtleitenden Rohrleitungsstück zwei Elektroden angebracht, an denen die induzierte Spannung gemessen wird.

Bei kapazitiven Uroflowmetern, beispielsweise nach der DE-PS 25 00 094, wird eine Flüssigkeitsstandmessung in eine Kapazitätsänderung umgesetzt. Hierzu ist ein meist zylindrischer, elektrischer Kondensator in dem Auffanggefäß der Meßflüssigkeit angebracht. Mit der Füllhöhe ändert sich die Kapazität der Anordnung und ist somit ein Maß für den zeitabhängigen Füllstand und damit für die Volumenfunktion aus der durch elektrische Differentiation der Harnfluß ermittelt wird.

Aus der DE-AS 23 30 033 ist weiter eine Anordnung bekannt, bei der die elektrolytische Leitfähigkeit des Urins zur Messung des Flüssigkeitsstandes genutzt wird. Hierbei wird ein nicht isolierter Widerstand gegenüber einer äußeren Elektrode durch die leitfähige Flüssigkeit teilweise überbrückt bzw. kurzgeschlossen. Dabei ändert sich der Widerstand mit der Höhe des Flüssigkeitsniveaus und ist daher ein Maß für den Füllstand.

Der Nachteil der meisten auf elektrischem Wege arbeitenden Uroflowmeter besteht unter anderem darin, daß sie nach jeder Benutzung teils umständlich entleert und gereinigt werden müssen, wodurch sich Fehlerquellen ergeben können. Bei der genannten Widerstandsmessung können z.B. Widerstandswindungen der elektrischen Widerstände durch Harnkristalle überbrückt werden, was ebenso zu Meßfehlern führen kann, wie leitfähige Beläge, die sich zwischen den Meßelektroden bilden können. Uroflowmeter, die nach dem rotationsdynamischen Prinzip arbeiten, weisen neben Reinigungsproblemen noch den Nachteil auf, daß sie aufgrund der Verwendung eines Antriebsmotors für die Scheibe sowie deren Lagerung ziemlich aufwendig sind. Bei induktiven Durchflußmessern ist das Meßsignal relativ klein und der erforderliche starke Magnet relativ teuer.

Es ist daher die Aufgabe der Erfindung, eine Harndurchflußmeßeinrichtung zu schaffen, die funktionssicher zu betreiben ist und genaue Messungen ermöglicht sowie ohne Demontage zu reinigen ist.

Diese Aufgabe wird erfindungsgemäß dadurch gelöst, daß die Meßeinrichtung ein in ein WC-Becken einhängbares, sich an ein unten offenes Auffanggefäß und ein sich daran anschließendes, andernends offenes Rohr mit einem ersten Meßwertaufnehmer zur fortlaufenden Bestimmung der elektrischen Leitfähigkeit der durch das Rohr fließenden Flüssigkeit und diesem folgend mit einem zweiten Meßwertaufnehmer zur Bestimmung der spezifischen elektrischen Leitfähigkeit der Flüssigkeit umfaßt und daß eine Auswerteelektronik für Meßwertaufnehmer vorgesehen ist.

Die mit dieser Meßeinrichtung erzielbaren Vorteile bestehen insbesondere darin, daß eine Entleerung der Meßeinrichtung grundsätzlich nicht vorgenommen werden muß, da bei der erfindungsgemäßen Einrichtung kein Sammelgefäß benötigt wird. Die Reinigung kann ohne weiteres nach jeder Benutzung durch einfaches Durchspülen erfolgen, wodurch u.a. die Ablagerung von Harnkristallen vermieden wird.

Nach einer vorteilhaften Ausgestaltung der Erfindung können der erste Meßwertaufnehmer zwei mit der Oberfläche der Innenwandung des Rohres abschließende, in Distanz zueinander angeordnete Ringelelektroden und der zweite Meßwertaufnehmer eine Meßkammer, in der eine Zylinderringelektrode und zentral innerhalb dieser eine Stiftelektrode angeordnet sind, umfassen. Dabei kann das Rohr einen sich an das als Trichter ausgebildete Auffanggefäß anschließenden, geneigt verlaufenden Abschnitt aufweisen, der in einen im Winkel zu diesem angeordneten Endabschnitt übergeht, wobei der erste Meßwertaufnehmer dem geneigten Abschnitt und der zweite Meßwertaufnehmer dem Endabschnitt zugeordnet sein kann. In Verbindung mit einer dem ersten Meßwertaufnehmer vorgeordneten Laminierzone aus in Richtung der Achse des Rohres verlaufenden Nuten bzw. Stegen gelangt so die zu kontrollierende Flüssigkeit in laminarer Strömung in den Bereich des ersten Meßwertaufnehmers, der ungehindert durchflossen wird, so daß eine genaue Messung der elektrischen Leitfähigkeit der Flüssigkeit erfolgen kann.

Bei der Bestimmung der spezifischen elektrischen Leitfähigkeit durch den zweiten Meßwertaufnehmer,ist es erforderlich, daß dessen Meßkammer vollständig gefüllt ist. Das kann dadurch sichergestellt werden, daß die Meßkammer eine große Zulauföffnung, eine kleine Ablauföffnung sowie eine zusätzliche Überlauföffnung aufweist, so daß die zwischen den beiden Elektroden durchströmende Flüssigkeitsmenge konstant ist. Die Auswertung der durch die Meßwertaufnehmer ermittelten Werte wird durch die Auswerteelektronik erfolgen, die jeweils mindestens einen Prozessor, einen Funktionsspeicher, einen Meßwertspeicher und einen Offsetspeicher umfaßt.Schließlich kann jeder Meßwertaufnehmer eine eigene Spannungsversorgung aufweisen, so daß aufgrund der dadurch erreichbaren Entkoppelung der Systeme eventuelle Meßfehler durch Koppelwiderstände ausgeschlossen sind.

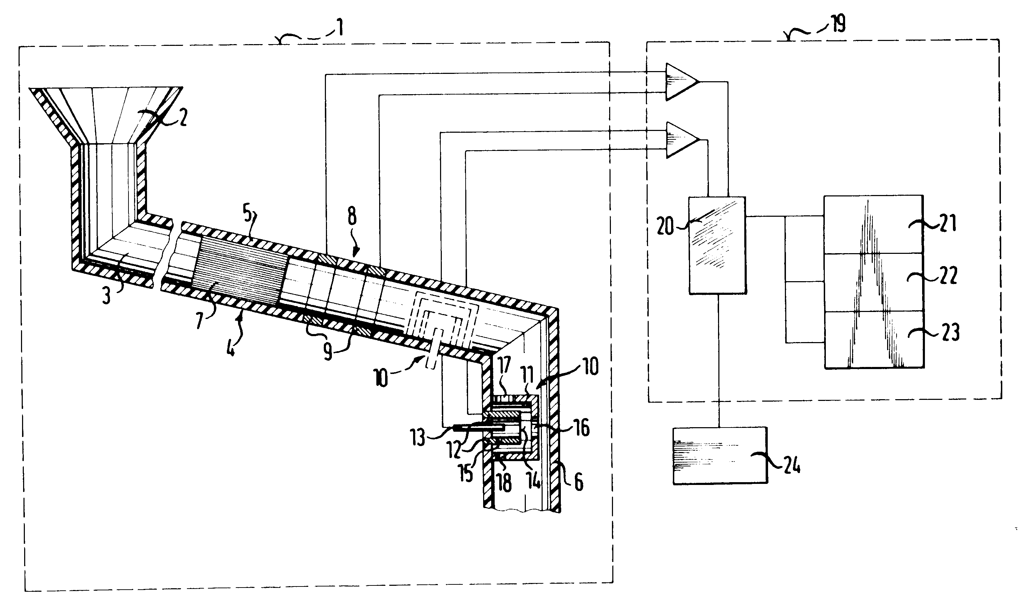

Die Erfindung ist nachstehend anhand eines in der Zeichnung dargestellten Ausführungsbeispieles näher erläutert.

Die mit dem Bezugszeichen 1 gekennzeichnete Meßeinrichtung zur Aufnahme der Meßwerte besteht im wesentlichen aus einem Auffangtrichter 2, der über einen Krümmerstutzen 3 mit seitlichem Ausgang in einen geneigt verlaufenden Abschnitt 5 eines Rohres 4 aus einem nichtleitenden Werkstoff, beispielsweise PVC, mündet, welches sich weiter in einen endseitig offenen Endabschnitt 6 fortsetzt. In dem geneigt verlaufenden Abschnitt 5 ist zunächst eine Laminierzone 7 angeordnet,die im wesentlichen aus in Richtung der Achse des Rohres 4 verlaufenden Nuten bzw. Stegen besteht. Der Laminierzone 7 ist ein erster Meßwertaufnehmer 8 nachgeordnet, der ebenfalls in dem geneigt verlaufenden Abschnitt 5 untergebracht ist und zwei mit der Oberfläche der Innenwandung des Rohres 4 abschließende, in Distanz zueinander angeordnete Ringelektroden 9 umfaßt. In dem Endabschnitt 6 des Rohres 4 ist ein zweiter Meßwertaufnehmer 10, bestehend aus einer Meßkammer 11 mit einer darin befindlichen Zylinderringelektrode 12 und einer in deren Zentrum angeordneten Stiftelektrode 13, angeordnet. Die Meßkammer 11 ist mit einer Zulauföffnung 17, einer dieser diametral gegenüberliegenden Ablauföffnung 18 und einer seitlichen Überlauföffnung 16 versehen. Die in der Meßkammer 11 angeordnete Zylinderringelektrode 12 weist gleichfalls eine große Zulauföffnung 14 und eine kleine Ablauföffnung 15 auf.

Die Meßwertaufnehmer 8 und 10 sind an die Eingänge einer Auswerteelektronik 19 angeschlossen, die mindestens einen Prozessor 20, einen Funktionsspeicher 21, einen Meßwertspeicher 22 und einen Offsetspeicher 23 umfaßt. Zur Ausgabe der Meßwerte findet bspw. ein Drucker 24 Verwendung.

Zur Aufzeichnung der Uroflowmetriekurve wird die Meßeinrichtung zweckmäßigerweise in ein WC-Becken eingehängt.

Der während der Miktion ausgeschiedene Urin gelangt über den Auffangtrichter 2 und den Krümmerstutzen 3 in den geneigt verlaufenden Abschnitt 5 des Rohres 4, wo die zunächst wirbelhafte Flüssigkeitsströmung durch die Laminierzone 7 in eine laminare Strömung umgewandelt wird. Dies ist vorteilhaft, um durch ein wirbelhaftes asymetrisches Strömungsprofil mögliche Meßfehler in dem folgenden Meßvorgang zu vermeiden. Bei der Durchströmung der Ringelektroden 9 des ersten Meßwertaufnehmers 8 ergibt sich durch den Urinstrom eine elektrisch leitfähige Verbindung, wobei die Leitfähigkeit davon abhängig ist, wieviel Flüssigkeit sich zwischen den Ringelektroden 9 befindet. Der fortlaufend gewonnene Wert der Leitfähigkeit ist somit ein Maß für den Durchfluß. Da der gemessene Leitfähigkeitswert auch von der spezifischen elektrischen Leitfähigkeit abhängt, wird dieser Wert durch den zweiten Meßwertaufnehmer 10 bestimmt. Dazu muß dessen Meßkammer 11 vollständig gefüllt sein. Das wird dadurch sichergestellt, daß die Flüssigkeit durch die vergleichsweise große Zulauföffnung 17 der Meßkammer 11 zugeleitet wird, wobei aufgrund der kleineren Ablauföffnung 18 nicht die gleiche Menge abfließen kann. Eventuell vorhandene Luftblasen sowie eine das Volumen der Meßkammer übersteigende Flüssigkeitsmenge können durch die Überlauföffnung 16 aus der Meßkammer 11 entweichen, so daß eine Verfälschung des Meßergebnisses durch Lufteinschlüsse sicher vermieden ist.

Bedingt durch die Geometrie des ersten Meßwertaufnehmers 8 sowie die Neigung des Rohrabschnittes 5, in dem dieser untergebracht ist, ist der Zusammenhang zwischen Leitfähigkeit und Durchfluß nicht linear. Dieser Zusammenhang ist in dem Funktionsspeicher 21 der Auswerteelektronik 19 gespeichert und wird von dieser durch Multiplikation der gemessenen Leitfähigkeitswerte mit dem Speicherinhalt berücksichtigt. Diese mathematischen Operationen werden alle von dem Prozessor 20 durchgeführt, durch den auch das Abspeichern der Meßwerte in dem Meßwertspeicher 23 und der Offsetwerte der Meßwertaufnehmer 8 und 10 in dem Offsetspeicher 23 erfolgt. Die Offsetwerte werden jeweils vor dem Einsatz der Meßeinrichtung, das heißt vor der Benetzung der Meßwertaufnehmer 8 und 10 bestimmt, wodurch sichergestellt wird, daß etwaige leitfähige Beläge, die mit der Zeit unter Umständen zwischen den Elektroden auftreten könnten, das Meßergebnis nicht verfälschen. Die entsprechende Berücksichtigung der Offsetwerte erfolgt durch den Prozessor 20 durch jeweilige Subtraktion derselben von den aktuellen Meßwerten. Über den Prozessor 20 werden auch die Ausgabe von Anstiegszeit, maximalem Fluß, durchschnittlichem Fluß, Volumen, Miktionszeit und Flußzeit sowie Durchflußrate gesteuert, wobei der Durchfluß entweder in Zahlenwerten oder als Fluß-Zeit-Diagramm mit dem Drucker 24 ausgedruckt werden kann.

In weiterer Ausbildung der beschriebenen Meßeinrichtung kann diese zur Erzeugung einer laminaren Strömung auch aus einer Vielzahl von Röhren aufgebaut werden, wobei der Durchmesser der Röhren möglichst klein, aber wiederum so groß zu wählen ist, daß ein Verbleiben von Flüssigkeit in den Röhren vermieden wird.

Weiter kann die Meßkammer 11 des zweiten Meßwertaufnehmers 10 unter Beibehaltung der großen Zulauföffnung 17 und der kleineren Ablauföffnung 18 anstelle der konzentrischen Anorndung der Zylinderringelektrode 12 und der Stiftelektrode 13 eine Elektrodenanordnung bspw. aus zwei Kontaktstiften oder dergleichen aufweisen.

## Patentansprüche

1. Meßeinrichtung (1) zur Harndurchflußmessung für die urologische Funktionsdiagnostik durch Messen des Harnflusses, wobei die Meßeinrichtung ein in ein WC-Becken einhängbares, sich an ein unten offenes Auffanggefäß (2) anschließendes, andernends offenes Rohr (4) mit einem ersten Meßwertaufnehmer (8) zur fortlaufenden Bestimmung der elektrischen Leitfähigkeit der durch das Rohr (4) fließenden Harnflüssigkeitsmenge und mit einem dem Aufnehmer (8) folgenden zweiten Meßwertaufnehmer (10) zur Bestimmung der spezifischen elektrischen Leitfähigkeit der Harnflüssigkeit umfaßt und wobei für die Werte der beiden Meßwertaufnehmer (8, 10) eine Auswerteelektronik (19) zur Ermittlung des Harndurchflusses vorgesehen ist.

2. Meßeinrichtung nach Anspruch 1, dadurch gekennzeichnet, daß der erste Meßwertaufnehmer (8) zwei mit der Oberfläche der Innenwandung des Rohres (4) abschließende und in Distanz zueinander angeordnete Ringelektroden (9) aufweist und daß der zweite Meßwertaufnehmer (10) aus einer Meßkammer (11) besteht, in der eine Zylinderringelektrode (12), die mit einer großen Zulauföffnung (14) und einer kleinen Ablauföffnung (15) versehen ist, und eine zentrale Stiftelektrode (13) angeordnet sind.

3. Meßeinrichtung nach Anspruch 1 oder 2, dadurch gekennzeichnet, daß das sich an das Auffanggefäß in Form eines Trichters (2) anschließende Rohr (4) geneigt verläuft und daß der geneigte Abschnitt (5) des Rohres (4) die beiden Meßwertaufnehmer (8,10) im Abstand zueinander aufweist.

4. Meßeinrichtung nach Anspruch 1 oder 2, dadurch gekennzeichnet, daß das Rohr (4) einen sich an das Auffanggefäß in Form eines Trichters (2) anschließenden und geneigt verlaufenden Abschnitt (5) aufweist, der in einen im Winkel zu diesem angeordneten Endabschnitt (6) übergeht, wobei der erste Meßwertaufnehmer (8) dem geneigt verlaufenden Abschnitt (5) und der zweite Meßwertaufnehmer (10) dem Endabschnitt (6) des Rohres (4) zugeordnet ist.

5. Meßeinrichtung nach einem der Ansprüche 1 bis 4, dadurch gekennzeichnet, daß dem ersten Meßwertaufnehmer (8) eine Zone (7) zum Erzeugen einer laminaren Strömung vorgeordnet ist, die im wesentlichen aus in Richtung der Achse des Rohres (4) verlaufenden Nuten bzw. Stegen besteht.

6. Meßeinrichtung nach einem der Ansprüche 1 bis 5, dadurch gekennzeichnet, daß die Meßkammer (11) des zweiten Meßwertaufnehmers (10) eine große Zulauföffnung (17), eine dieser gegenüber angeordnete kleinere Ablauföffnung (18) sowie eine Überlauföffnung (16) aufweist.

7. Meßeinrichtung nach einem der Ansprüche 1 bis 6, dadurch gekennzeichnet, daß die mit den Meßwertaufnehmern (8,10) in Verbindung stehende Auswerteelektronik (19) mindestens jeweils einen Prozessor (20), einen Funktionsspeicher (21), einen Meßwertspeicher (22) und einen Offsetspeicher (23) umfaßt.

8. Meßeinrichtung nach einem der Ansprüche 1 bis 7, dadurch gekennzeichnet, daß jeder Meßwertaufnehmer (8,10) eine eigene Spannungsversorgung aufweist.

## Claims

1. Measuring device (1) for urine throughflow measurement for urological function diagnosis by measuring urine flow, wherein the measuring device includes a pipe (4) which can be suspended in a toilet bowl and adjoins an open-bottomed collecting vessel (2) and is open at the other end, with a first transducer (8) for continuous determination of the electrical conductivity of the quantity of urine liquid flowing through the pipe (4), and with a second transducer (10) following the transducer (8) for determining the specific electrical conductivity of the urine liquid, and wherein an electronic analyser (19) for determining urine throughflow is provided for the values of the two transducers (8, 10).

2. Measuring device according to claim 1, characterised in that the first transducer (8) comprises two ring electrodes (9) sealing with the surface of the inner wall of the pipe (4) and arranged at a distance from each other and in that the second transducer (10) consists of a measuring chamber (11) in which are arranged a cylinder ring electrode (12), which is provided with a large inlet opening (14) and a small outlet opening (15), and a central pin electrode (13).

3. Measuring device according to claim 1 or 2, characterised in that the pipe (4) in the form of a funnel (2) adjoining the collecting vessel extends obliquely and in that the inclined section (5) of the pipe (4) comprises the two transducers (8, 10) spaced from each other.

4. Measuring device according to claim 1 or 2, characterised in that the pipe (4) comprises a section (5) which adjoins the collecting vessel in the form of a funnel (2) and extends obliquely and which merges with an end section (6) arranged at an angle thereto, wherein the first transducer (8) is associated with the section (5) extending obliquely and the second transducer (10) is associated with the end section (6) of the pipe (4).

5. Measuring device according to any of claims 1 to 4, characterised in that in front of the first transducer (8) is arranged a zone (7) for producing a laminar stream which essentially consists of channels or ridges extending in the direction of the axis of the pipe (4).

6. Measuring device according to any of claims 1 to 5, characterised in that the measuring chamber (11) of the second transducer (10) comprises a large inlet opening (17), a smaller outlet opening (18) arranged opposite the latter, and also an overflow opening (16).

7. Measuring device according to any of claims 1 to 6, characterised in that the electronic analyser (19) connected to the transducers (8, 10) includes at least in each case a processor (20), a function memory (21), a measured value memory (22) and an offset memory (23).

8. Measuring device according to any of claims 1 to 7, characterised in that each transducer (8, 10) comprises its own voltage supply.

## Revendications

1. Mécanisme de mesure (1) destiné à la mesure du débit urinaire pour le diagnostic de la fonction urologique par la mesure de l'écoulement urinaire, dans lequel le mécanisme de mesure comprend un tube (4) apte à être suspendu dans une cuvette de W-C, qui vient se raccorder à un récipient de réception (2) ouvert vers le bas et qui est ouvert à son autre extrémité, muni d'un premier transducteur (8) pour la détermination en continu de la conductibilité électrique de la quantité de liquide urinaire s'écoulant à travers le tube (4) et d'un second transducteur (10) qui suit le transducteur (8) pour la détermination de la conductibilité électrique spécifique du liquide urinaire, et dans lequel on prévoit, pour les valeurs des deux transducteurs (8, 10), une électronique d'évaluation (19) pour déterminer le débit urinaire.

2. Mécanisme de mesure selon la revendication 1, caractérisé en ce que le premier transducteur (8) présente deux électrodes annulaires (9) qui se terminent avec la surface de la paroi interne du tube (4) et qui sont disposées à distance mutuelle, et en ce que le second transducteur (10) est constitué d'une chambre de mesure (11) dans laquelle sont disposées une électrode cylindrique annulaire (12), qui est munie d'une grande ouverture d'entrée (14) et d'une petite ouverture de sortie (15), et une électrode centrale (13) en forme de broche.

3. Mécanisme de mesure selon la revendication 1 ou 2, caractérisé en ce que le tube (4) qui se raccorde au récipient de réception sous la forme d'un entonnoir (2), s'étend en inclinaison, et en ce que la section inclinée (5) du tube (4) présente les deux transducteurs (8, 10) à distance l'un de l'autre.

4. Mécanisme de mesure selon la revendication 1 ou 2, caractérisé en ce que le tube (4) présente une section (5) s'étendant en inclinaison et se raccordant au récipient de réception sous la forme d'un entonnoir (2), qui se transforme en une section terminale (6) disposée en formant un angle par rapport à la première, dans lequel le premier transducteur (8) est attribué à la section (5) s'étendant en inclinaison et le second transducteur (10) est attribué à la section terminale (6) du tube (4).

5. Mécanisme de mesure selon l'une quelconque des revendications 1 à 4, caractérisé en ce qu'une zone (7) pour obtenir un écoulement laminaire est disposée en amont du premier transducteur (8), qui est constituée essentiellement de rainures, respectivement de nervures, s'étendant dans la direction de l'axe du tube (4).

6. Mécanisme de mesure selon l'une quelconque des revendications 1 à 5, caractérisé en ce que la chambre de mesure (11) du second transducteur (10) présente une grande ouverture d'entrée (17), une ouverture de sortie (18) plus petite disposée à l'opposé, ainsi qu'une ouverture de trop-plein (16).

7. Mécanisme de mesure selon l'un quelconque des revendications 1 à 6, caractérisé en ce que l'électronique d'évaluation (19) qui se trouve en liaison avec les transducteurs (8, 10) comprend au moins respectivement un processeur (20), une mémoire de fonctions (21), une mémoire de valeurs de mesures (22) et une mémoire offset (23).

8. Mécanisme de mesure selon l'une quelconque des revendications 1 à 7, caractérisé en ce que chaque transducteur (8, 10) présente une alimentation en tension qui lui est propre.
